# EUROPEAN PATENT APPLICATION

(11) **EP 4 177 334 A1**
(43) Date of publication of application: **10.05.2023**
(21) Application number: 21950816.5
(22) Date of filing: 30.11.2021
(51) Int. Cl.: C12M 1/40, C12M 1/02, C12M 1/12, C12M 1/00

(54) **PROTEIN RECOVERY SYSTEM AND METHOD IN PRODUCTION PROCESS OF ULTRA-HIGH MALTOSE SYRUP**

(30) Priority: 21.07.2021 CN 202110821695
(71) Applicant: Zhejiang Huakang Pharmaceutical Co., Ltd., Quzhou, Zhejiang 324302 (CN)
(72) Inventor: CHENG, Xinping, Quzhou, Zhejiang 324302 (CN); CHEN, Shengrong, Quzhou, Zhejiang 324302 (CN); CHENG, Yuqing, Quzhou, Zhejiang 324302 (CN); HAN, Yuanlong, Quzhou, Zhejiang 324302 (CN); LIAO, Chengjun, Quzhou, Zhejiang 324302 (CN); LUO, Jiaxing, Quzhou, Zhejiang 324302 (CN); WANG, Xiaobin, Quzhou, Zhejiang 324302 (CN)
(74) Representative: Sun, Yiming
(86) International application number: PCT/CN2021/134597
(87) International publication number: WO 2023/000578

(57) **Abstract**

The present invention provides a system for recovering protein in a production process of an ultrahigh maltose syrup, including a saccharification tank, an enzyme preparation tank, a first plate heat exchanger, a second plate heat exchanger, a plate and frame filter, a buffer tank and a rotary drum filter. The present invention further provides a method of recovering protein by using the system. After the sugar liquid in the saccharification tank is stood, the protein floats at the upper part of the saccharification tank and the lower liquid is clear and transparent and thus the sugar liquid can be directly filtered. When the saccharification tank is discharged, the lower liquid is firstly discharged with the remaining liquid being bottoms containing protein. During a production process, enzymatic hydrolysis is performed for the sugar liquid containing protein before filtration to improve the filtration effect of the sugar liquid. Assisted by the plate and frame filter, protein can be recovered. The present invention improves the economic benefits, reduces the production costs, and thus solves the problem of difficulty in recovering protein in a production process of an ultrahigh maltose syrup.

## Description

### TECHNICAL FIELD

The present invention relates to the field of maltose syrup production technologies, and in particular to a system and a method for recovering protein in a production process of an ultrahigh maltose syrup.

### BACKGROUND

Ultrahigh maltose syrup is a syrup with a maltose content above 90%, which is produced with a corn starch as a raw material and is one of starch sugars. This syrup is hydrogenated and finally used for production of crystalline maltitol.

Starch contains some protein (corn starch contains 0.3% protein) which will be flocculated out after being liquefied. In a glucose production process, protein can be recovered through filtration by using a plate and frame filter (plate and frame filtration) and the recovered coarse protein can be processed into fodder, thus increasing its value.

But, in a production process of an ultrahigh maltose syrup, protein is difficult to recover for the following reason: in order to obtain higher content maltose, a liquefaction DE value is controlled to be less than 4 relative to the glucose production. In a case of low DE value, a liquefaction liquid to be saccharified contains much macromolecular dextrin as well as some oil fats. During recovery, it is difficult for the plate and frame filter to perform filtration and the running time is only 1/4 of the time for filtering glucose. Further, frequent switching of the plate and frame filter will affect continuous production. Further, syrup loss occurs due to wet protein residues.

The current process is to directly remove protein by using a plate and frame filter press or a rotary drum filter (mainly with diatomite as filter aid). Because protein and diatomite are mixed together after filtration, the protein cannot be recovered for use. Further, diatomite consumption increases costs. The patent with the publication number CN101684504A discloses that the method is used to remove insoluble substances such as protein in a sugar liquid. The patent with the publication number CN107090012A discloses that a high maltose syrup (content 70 to 80%) is produced by using potato and protein is recovered by using a plate and frame filtration process, which indicates that the plate and frame filter can be used for protein recovery. But, because the high maltose syrup is produced using potato as raw material in the patent, the liquefaction and saccharification processes are different from those of producing an ultrahigh maltose syrup and have no difficulty in filtration. In comparison, the problem is present in the process where an ultrahigh maltose syrup is produced using a corn starch. Therefore, improvement is to be made.

### SUMMARY

The present invention provides a system and a method for recovering protein in a production process of an ultrahigh maltose syrup to solve the shortcomings of the prior arts.
The present invention is achieved in the following technical solution.

There is provided a system for recovering protein in a production process of an ultrahigh maltose syrup, including a saccharification tank, an enzyme preparation tank, a first plate heat exchanger, a second plate heat exchanger, a plate and frame filter, a buffer tank and a rotary drum filter. The enzyme preparation tank is communicated with a liquid inlet end of the saccharification tank through a pipe, and the liquid inlet end of the saccharification tank is communicated with a liquefaction liquid to be saccharified and a saccharifying enzyme through a pipe respectively. A liquid outlet end of the saccharification tank is communicated with an inlet end of the first plate heat exchanger through a pipe, and an outlet end of the first plate heat exchanger is communicated with the liquid inlet end of the saccharification tank through a pipe. The liquid outlet end of the saccharification tank is further communicated with an inlet end of the second plate heat exchanger through a pipe, and an outlet end of the second plate heat exchanger is communicated with an inlet end of the plate and frame filter through a pipe. Residues filtered by the plate and frame filter contain protein to be recovered. A residue outlet end of the plate and frame filter is communicated with an inlet end of a spiral conveyor through a pipe. The residues containing protein is transferred by the spiral conveyor for recycling. A liquid outlet end of the plate and frame filter is communicated with a liquid inlet end of the buffer tank through a pipe, a liquid outlet end of the buffer tank is communicated with the rotary drum filter through a pipe, and a liquid outlet end of the rotary drum filter is communicated with a subsequent working procedure.

There is provided a method of recovering protein in a production process of an ultrahigh maltose syrup, which uses the above system for recovering protein in a production process of an ultrahigh maltose syrup, where one or more saccharification tanks are used.

When one saccharification tank is used, the method of recovering protein in a production process of an ultrahigh maltose syrup includes the following steps:
(a) opening a feed valve a of the 1# saccharification tank, start stirring and allowing the liquefaction liquid to be saccharified to enter the 1# saccharification tank, and adding the saccharifying enzyme at the same time; when the liquid level of the 1# saccharification tank is 100%, closing the feed valve a;
(b) after completion of the step (a), performing stirring reaction for the 1# saccharification tank until the stirring reaction ends, and standing the 1# saccharification tank to make protein float at an upper part of the 1# saccharification tank as possible;
(c) after completion of the step (b), opening a discharge valve b of the 1# saccharification tank and starting the sugar liquid pump to enable the syrup to pass through the second plate heat exchanger to cool down through heat exchange with an evaporation feed and enter the plate and frame filter for filtration, conveying a filtrate into the buffer tank and then into the rotary drum filter for filtration to obtain a rotary drum filtrate, and then performing decolorization, ion exchange and evaporation for the rotary drum filtrate to finally obtain a finished ultrahigh maltose syrup;
(d) when the remaining liquid level of the 1# saccharification tank is 10-15%, closing the discharge valve b to stop discharging;
(e) after completion of the step (d), weighing a lysophospholipase into the enzyme preparation tank, starting a diaphragm pump and opening a valve g to convey the lysophospholipase into the 1# saccharification tank and closing the valve g;
(f) after completion of the step (e), performing stirring reaction for the 1# saccharification tank until the stirring reaction ends;
(g) after completion of the step (f), opening a valve c and a valve h and starting the circulation pump, enabling the syrup in the 1# saccharification tank to run through the first plate heat exchanger for being heated cyclically; when an entire temperature of the 1# saccharification tank reaches a preset temperature, stopping cyclic heating;
(h) after completion of the step (g), weighing a high-temperature-resistant α-amylase into the enzyme preparation tank, starting the diaphragm pump and opening the valve g to convey the high-temperature-resistant α-amylase into the 1# saccharification tank, and closing the valve g;
(i) after completion of the step (h), performing stirring reaction for the 1# saccharification tank until the stirring reaction ends;
(j), after completion of the step (i), opening the discharge valve b of the 1# saccharification tank, enabling the sugar liquid containing protein to run through the second plate heat exchanger to cool down through heat exchange with the evaporation feed, and enter the plate and frame filter for filtration, retaining protein in the plate and frame filter, and conveying a filtrate into the buffer tank, and then into the rotary drum filter for filtration to obtain a rotary drum filtrate, and performing decolorization, ion exchange and evaporation for the rotary drum filtrate to finally obtain a finished ultrahigh maltose syrup, discharging bottoms completely to the liquid level 0%;
(k) after completion of the step (j), closing the discharge valve b and after the protein is dried, discharging the protein to the spiral conveyor below the plate and frame filter for recovery;
when a plurality of saccharification tanks, for example, two saccharification tanks, are used, the method of recovering protein in a production process of an ultrahigh maltose syrup includes the following steps:
(A) opening the feed valve a of the 1# saccharification tank, start stirring, and allowing the liquefaction liquid to be saccharified to enter the 1# saccharification tank and adding the saccharifying enzyme at the same time; when the liquid level of the 1# saccharification tank is 100%, closing the feed valve a and opening the valve d to allow the liquefaction liquid to be saccharified and the saccharifying enzyme to enter the 2# saccharification tank;
(B) after completion of the step (A) and closing of the feed valve a, performing stirring reaction for the 1# saccharification tank until the stirring reaction ends, and standing the 1# saccharification tank to make protein float at an upper part of the 1# saccharification tank as possible; and performing same operations for the 2# saccharification tank;
(C), after completion of the step (B) and stooding of the 1# saccharification tank, opening the discharge valve b of the 1# saccharification tank and starting the sugar liquid pump to enable the syrup to pass through the second plate heat exchanger to cool down through heat exchange with an evaporation feed and enter the plate and frame filter for filtration, conveying a filtrate into the buffer tank and then into the rotary drum filter for filtration to obtain a rotary drum filtrate, and then performing decolorization, ion exchange and evaporation for the rotary drum filtrate to finally obtain a finished ultrahigh maltose syrup;
(D) when the remaining liquid level of the 1# saccharification tank is 10-15%, stop discharging; firstly closing the discharge valve b and then opening a valve e, and performing the same discharge operations to the 2# saccharification tank as the 1# saccharification tank; during a discharge process of the 2# saccharification tank, performing enzyme preparation reaction treatment for the bottoms of the 1# saccharification tank by referring to the following specific operations shown in the steps (E), (F), (G), (H) and (I); performing the same operations for the bottoms of the 2# saccharification tank for enzyme preparation reaction treatment;
(E) weighing a lysophospholipase into the enzyme preparation tank, starting the diaphragm pump and opening the valve g to convey the lysophospholipase into the 1# saccharification tank and closing the valve g;
(F) after completion of the step (E), performing stirring reaction for the 1# saccharification tank until the stirring reaction ends;
(G) after completion of the step (F), opening the valve c and the valve h and starting the circulation pump, enabling the syrup in the 1# saccharification tank to run through the first plate heat exchanger for being heated cyclically; when an entire temperature of the 1# saccharification tank reaches a preset temperature, stopping cyclic heating;
(H) after completion of the step (G), weighing a high-temperature-resistant α-amylase into the enzyme preparation tank, starting the diaphragm pump and opening the valve g to convey the high-temperature-resistant α-amylase into the 1# saccharification tank, and closing the valve g after completing the conveying;
(I) after completion of the step (H), performing stirring reaction for the 1# saccharification tank until the stirring reaction ends with the bottoms to be discharged by referring to the specific operations shown in the steps (J) and (K); performing the same operations to discharge the bottoms of the 2# saccharification tank;
(J) when discharging the bottoms of the 1# saccharification tank, closing the discharge valves of other tanks, and opening the discharge valve b of the 1# saccharification tank, enabling the sugar liquid containing protein to run through the second plate heat exchanger to cool down through heat exchange with the evaporation feed, and enter the plate and frame filter for filtration, retaining protein in the plate and frame filter, and conveying a filtrate into the buffer tank, and then into the rotary drum filter for filtration to obtain a rotary drum filtrate, and performing decolorization, ion exchange and evaporation for the rotary drum filtrate to finally obtain a finished ultrahigh maltose syrup, discharging the bottoms completely to the liquid level 0%;
(K) after completion of the step (J), closing the discharge valve b and opening the valve e, and continuing discharging other saccharified syrup; being ready to feed the 1# saccharification tank at the same time; after the protein is dried, discharging the protein to the spiral conveyor below the plate and frame filter for recovery.

The present invention has the following beneficial effects.
1. After the syrup is stood, the protein floats at the upper part of the saccharification tank and the lower liquid is clear and transparent and thus the syrup can be directly filtered. When the saccharification tank is discharged, the lower liquid is firstly discharged with the remaining liquid being bottoms containing protein. There are several reasons why the protein-containing syrup is difficult to filter. One of the reasons is that the protein contains much macromolecular dextrin and oil fats. Enzyme preparation (lysophospholipase and high-temperature-resistant α-amylase) treatment is performed for this part of protein-containing sugar liquid. The process flows and control parameters are designed based on the reaction conditions of the enzyme preparations such that the enzyme preparations can better decompose the macromolecular dextrin and oil fat so as to improve the filtration effect. Assisted by the plate and frame filter, the protein can be recovered by filtration. Thus, the problem of the difficulty in recovering protein in a production process of an ultrahigh maltose syrup can be solved. At the time of discharge, heat exchange can be performed with the evaporation feed to improve the temperature of the evaporation feed and avoid thermal energy loss.
2. The present invention has the following beneficial effects.
   1) Recovery of protein: 0.015 to 0.02 tons of protein can be recovered from each dry basis ton of syrup, with recovery cost being 15 to 20 Yuan/dry basis ton.
   2) Reduction of diatomite consumption: For each dry basis ton of syrup, diatomite cost can be saved at the rate of 25Yuan/dry basis ton. The increased costs include the costs of power consumption, enzyme preparations and residue discharge, which are about 12 Yuan/dry basis ton in total. Entirely, for each dry basis ton of syrup, about 30 Yuan/dry basis ton can be saved.
   3) The syrup quality is improved, the hydrogenation efficiency is increased, the crystallization yield of the maltitol is increased, but the production costs are reduced.

### BRIEF DESCRIPTION OF THE DRAWINGS

FIG. 1 is a principle schematic diagram illustrating a system for recovering protein in a production process of an ultrahigh maltose syrup according to the present invention.
FIG. 2 is a principle schematic diagram illustrating an existing production process of a maltose syrup.

### DETAILED DESCRIPTION OF THE EMBODIMENTS

In order to make the technical problems to be solved, the technical solutions and the beneficial effects of the present invention clearer, the present invention will be further detailed in combination with drawings and embodiments. It should be understood that the specific embodiments described herein are used only to interpret the present invention rather than limit the present invention.

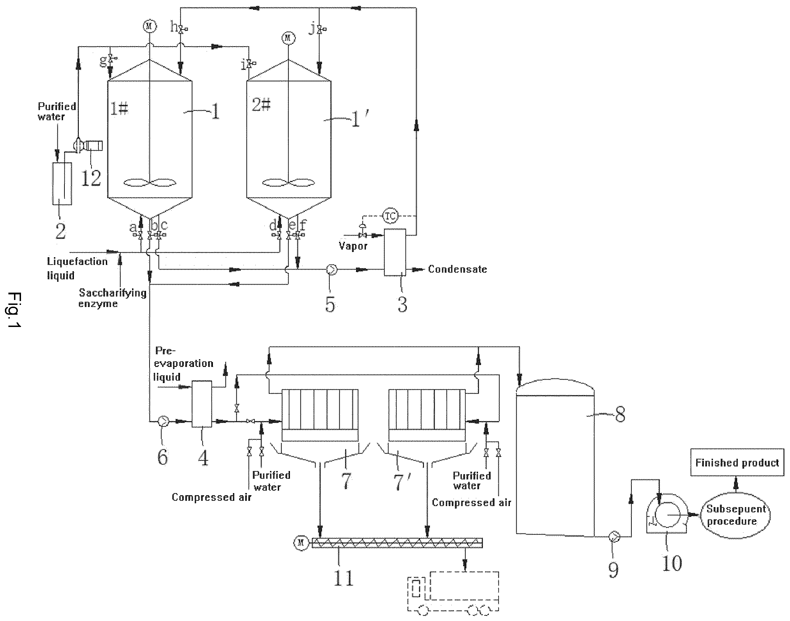

By referring to FIG. 1, a preferred embodiment of a system for recovering protein in a production process of an ultrahigh maltose syrup according to the present invention includes a saccharification tank 1, an enzyme preparation tank 2, a first plate heat exchanger 3, a second plate heat exchanger 4, a circulation pump 5, a sugar liquid pump 6, a plate and frame filter 7, a buffer tank 8, a syrup pump 9, and a rotary drum filter 10. The direction indicated by the arrow in the drawing is a flow or injection direction of materials (including solid, liquid and gas) in the system.

The enzyme preparation tank 2 is communicated with a liquid inlet end of the saccharification tank 1 through a pipe. The liquid inlet end of the saccharification tank 1 is communicated with a liquefaction liquid to be saccharified and a saccharifying enzyme through a pipe respectively. A liquid outlet end of the saccharification tank 1 is communicated with the circulation pump 5 and an inlet end of the first plate heat exchangers in sequence through a pipe. An outlet end of the first plate heat exchanger 3 is communicated with the liquid inlet end of the saccharification tank 1 through a pipe. The first plate heat exchanger 3 heats the flowing-through sugar liquid to a preset temperature by heat exchange.

The liquid outlet end of the saccharification tank 1 is further communicated with the sugar liquid pump 6 and an inlet end of the second plate heat exchanger 4 in sequence through a pipe. An outlet end of the second plate heat exchanger 4 is communicated with an inlet end of the plate and frame filter 7 through a pipe. The syrup cooled down through heat exchange with the second plate heat exchanger 4 enters the plate and frame filter 7 for filtration to filter out protein in the sugar liquid. The inlet end of the plate and frame filter 7 is further communicated with compressed air and purified water through a pipe respectively.

Residues filtered by the plate and frame filter 7 contain protein to be recovered and a residue outlet end of the plate and frame filter 7 is communicated with an inlet end of a spiral conveyor 11 through a pipe. The residues containing protein is transferred by the spiral conveyor for recycling. As shown in the drawing, the protein is transferred by a vehicle. A liquid outlet end of the plate and frame filter 7 is communicated with a liquid inlet end of the buffer tank 8 through a pipe, and a liquid outlet end of the buffer tank 8 is communicated with the syrup pump 9 and the rotary drum filter 10 in sequence through a pipe. A liquid outlet end of the rotary drum filter 10 is communicated with a subsequent working procedure. The subsequent working procedure includes performing decolorization, ion exchange and evaporation for the syrup obtained from the liquid outlet end of the rotary drum filter 10 to finally obtain a finished ultrahigh maltose syrup.

The circulation pump 5 is disposed on the pipe communicating the first plate heat exchanger 3 with the liquid outlet end of the saccharification tank 1, the sugar liquid pump 6 is disposed on the pipe communicating the second plate heat exchanger 4 with the liquid outlet end of the saccharification tank 1, and the syrup pump 9 is disposed on the pipe communicating the rotary drum filter 10 with the liquid outlet end of the buffer tank 8.

As shown in FIG. 1, the system displays two saccharification tanks: a 1# saccharification tank 1 and a 2# saccharification tank 1'as well as two plate and frame filters 7 and 7'.

The plate and frame filter 7 in the present invention is not added with filter aid or adsorbant. The rotary drum filter 10 uses diatomite as filter aid.

With reference to FIG. 1, the present invention further provides a method of recovering protein in a production process of an ultrahigh maltose syrup, which uses the above system for recovering protein in a production process of an ultrahigh maltose syrup, where one or more saccharification tanks are used.

When one saccharification tank is used, the method of recovering protein in a production process of an ultrahigh maltose syrup comprises the following steps:
(a) A feed valve a of the 1# saccharification tank 1 is opened, stirring is started and the liquefaction liquid to be saccharified with a temperature of 58 to 61 °C, a pH of 5.0 to 5.4 and a DE value of 3.0 to 4.0 is allowed to enter the 1# saccharification tank 1, and the saccharifying enzyme is added at the same time; when the liquid level of the 1# saccharification tank 1 is 100%, the feed valve a is closed.

The saccharifying enzyme used in the following embodiments may includeβ-amylase (Maiteer biology M-100), pullulanase (novozymes Promozyme D6), maltogenic amylase (novozymes Maltogenase 2XL) which are blended at a weight ratio of 1:1:1.
(b) After completion of the step (a), stirring reaction is performed for the 1# saccharification tank for 40-45h, and the 1# saccharification tank 1 is stood for ≥5h to make protein float at an upper part of the 1# saccharification tank 1 as possible. The reaction time is determined based on an enzyme addition amount.
(c) After completion of the step (b), a discharge valve b of the 1# saccharification tank 1 is opened and the sugar liquid pump 6 is started to enable the sugar liquid to pass through the second plate heat exchanger 4 (2# plate heat exchanger)to cool down through heat exchange with an evaporation feed (pre-evaporation liquid) and enter the plate and frame filter 7 for filtration, a filtrate is conveyed into the buffer tank 8 and then into the rotary drum filter 10 for filtration to obtain a rotary drum filtrate, and then decolorization, ion exchange and evaporation are performed for the rotary drum filtrate to finally obtain a finished ultrahigh maltose syrup.
(d) When the remaining liquid level of the 1# saccharification tank 1 is 10-15%, the discharge valve b is closed to stop discharging. Based on production experiences, the syrup of 15% liquid level contains greater than 98% of protein.
(e) After completion of the step (d), a lysophospholipase is weighed into the enzyme preparation tank 2 based on 0.16 to 0.22kg/dry basis ton, the diaphragm pump 12 is started and a valve g is opened to convey the lysophospholipase into the 1# saccharification tank 1 and then the valve g is closed.

When the enzyme preparation tank 2 and its pipe are to be cleaned, water is added to the enzyme preparation tank 2 (the volume of the added water must be greater than or equal to a volume of the conveying pipe), and pumped by the diaphragm pump 12 to the 1# saccharification tank 1 and water is further added (the volume of the added water must be greater than or equal to a volume of the conveying pipe) and pumped by the diaphragm pump 12 to the 1# saccharification tank 1 for cleaning the enzyme preparation tank 2 and the enzyme preparation pipe.

The lysophospholipase used in the following embodiments is novozymes Finizym^{®} W lysophospholipase.
(f) After completion of the step (e), stirring reaction is performed for the 1# saccharification tank 1 for ≥2h and stirring is stopped after the reaction.
(g) After completion of the step (f), a valve c and a valve h are opened, and the circulation pump 5 is started to enable the sugar liquid in the 1# saccharification tank 1 to run through the first plate heat exchanger 3 (1# plate heat exchanger) for being heated cyclically. An openness of a vapor valve is adjusted and a discharge temperature of the 1# plate heat exchanger is set to 95 to 97°C. When an entire temperature of the 1# saccharification tank 1 reaches a preset temperature (optimal enzymatic hydrolysis temperature for the high-temperature-reistantα- amylase), cyclic heating is stopped.
(h) After completion of the step (g), a high-temperature-resistant α-amylase is weighed into the enzyme preparation tank 2 at the rate of 0.36-0.44kg/dry basis ton, the diaphragm pump 12 is started and the valve g is opened to convey the high-temperature-resistant α-amylase into the 1# saccharification tank 1, and the valve g is closed after conveying is completed.

When the enzyme preparation tank 2 and its pipe are to be cleaned, water is added to the enzyme preparation tank 2 (the volume of the added water must be greater than or equal to a volume of the conveying pipe), and pumped by the diaphragm pump 12 to the 1# saccharification tank 1 and water is further added (the volume of the added water must be greater than or equal to a volume of the conveying pipe) and pumped by the diaphragm pump 12 to the 1# saccharification tank 1 for cleaning the enzyme preparation tank 2 and the enzyme preparation pipe.

The high-temperature-resistant α-amylase used in the following embodiment is Dupont SPEZYME^{®} POWERLIQ with its activity ≥30099LU/g.
(i) After completion of the step (h), stirring reaction is performed for the 1# saccharification tank 1 for ≥4h and stirring is stopped after the reaction.
(j) After completion of the step (i), the discharge valve b of the 1# saccharification tank is opened, the sugar liquid containing protein is enabled to run through the second plate heat exchanger to cool down through heat exchange with the evaporation feed (pre-evaporation liquid), and enter the plate and frame filter 7 for filtration, protein is retained in the plate and frame filter 7, and a filtrate is conveyed into the buffer tank 8, and then into the rotary drum filter 10 for filtration to obtain a rotary drum filtrate, decolorization, ion exchange and evaporation are performed for the rotary drum filtrate to finally obtain a finished ultrahigh maltose syrup, and bottoms are completely discharged to the liquid level 0%.
(k) After completion of the step (j), the discharge valve b is closed, and air ejection, water ejection and air ejection are performed on the plate and frame filter 7 for 10-15min, 2-5min and 15-20min respectively. The above times may be adjusted based on wetness of the protein residues. After the protein is dried, the protein is discharged to the spiral conveyor 11 below the plate and frame filter 7 for recovery.

During a filtration process of the plate and frame filter 7, when a flow rate is not satisfied or a pressure is >0.4MPa, the plate and frame filter 7 is to be changed.

When a plurality of saccharification tanks, for example, two saccharification tanks (1# saccharification tank 1 and 2# saccharification tank 1'), are used, the method of recovering protein in a production process of an ultrahigh maltose syrup inlcudes the following steps.
(A) A feed valve a of the 1# saccharification tank 1 is opened, stirring is started and the liquefaction liquid to be saccharified with a temperature of 58 to 61°C, a pH of 5.0 to 5.4 and a DE value of 3.0 to 4.0 is allowed to enter the 1# saccharification tank 1, and the saccharifying enzyme is added at the rate of 0.30 to 0.35kg /dry basis ton at the same time. When the liquid level of the 1# saccharification tank 1 is 100%, the feed valve a is closed and the valve d is opened and the liquefaction liquid to be saccharified and the saccharifying enzyme are allowed to enter the 2# saccharification tank.
(B) After completion of the step (A) and closing of the feed valve a, stirring reaction is performed for the 1# saccharification tank 1 for 40-45h, and the 1# saccharification tank 1 is stood for ≥5h to make protein float at an upper part of the 1# saccharification tank 1 as possible. Same operations are performed for the 2# saccharification tank 1'. The reaction time is determined based on an enzyme addition amount.
(C) After completion of the step (b), when the 1# saccharification tank 1 is stood for ≥5h, a discharge valve b of the 1# saccharification tank 1 is opened and the sugar liquid pump 6 is started to enable the sugar liquid to pass through the second plate heat exchanger 4 to cool down through heat exchange with an evaporation feed (pre-evaporation liquid) and enter the plate and frame filter 7 for filtration, a filtrate is conveyed into the buffer tank 8 and then into the rotary drum filter 10 for filtration to obtain a rotary drum filtrate, and then decolorization, ion exchange and evaporation are performed for the rotary drum filtrate to finally obtain a finished ultrahigh maltose syrup.
(D) When the remaining liquid level of the 1# saccharification tank 1 is 10-15%, the syrup of 15% liquid level contains greater than 98% of protein based on production experiences and the discharge is stopped. Firstly the discharge valve b is closed and then a valve e is opened, and the same discharge operations are performed to the 2# saccharification tank 1' as the 1# saccharification tank 1. During a discharge process of the 2# saccharification tank 1', enzyme preparation reaction treatment is performed for the bottoms of the 1# saccharification tank 1 by referring to the following specific operations shown in the steps (E), (F), (G), (H) and (I). The same operations are performed for the bottoms of the 2# saccharification tank 1' for enzyme preparation reaction treatment.
(E) A lysophospholipase is weighed into the enzyme preparation tank based on 0.16 to 0.22kg/dry basis ton, the diaphragm pump 12 is started and a valve g is opened to convey the lysophospholipase into the 1# saccharification tank 1 and then the valve g is closed after the conveying is completed.

When the enzyme preparation tank 2 and its pipe are to be cleaned, water is added to the enzyme preparation tank 2 (the volume of the added water must be greater than or equal to a volume of the conveying pipe), and pumped by the diaphragm pump 12 to the 1# saccharification tank 1 and water is further added (the volume of the added water must be greater than or equal to a volume of the conveying pipe) and pumped by the diaphragm pump 12 to the 1# saccharification tank 1 for cleaning the enzyme preparation tank 2 and the enzyme preparation pipe.
(F) After completion of the step (E), stirring reaction is performed for the 1# saccharification tank 1 for ≥2h and stirring is stopped after the reaction.
(G) After completion of the step (F), a valve c and a valve h are opened, and the circulation pump 5 is started to enable the sugar liquid in the 1# saccharification tank 1 to run through the first plate heat exchanger for being heated cyclically. An openness of a vapor valve is adjusted and a discharge temperature of the 1# plate heat exchanger is set to 95 to 97°C. When an entire temperature of the 1# saccharification tank 1 reaches a preset temperature (optimal enzymatic hydrolysis temperature for the high-temperature-resistant α- amylase), cyclic heating is stopped.
(H) After completion of the step (G), a high-temperature-resistant α-amylase is weighed into the enzyme preparation tank at the rate of 0.36-0.44kg/dry basis ton, the diaphragm pump 12 is started and the valve g is opened to convey the high-temperature-resistant α-amylase into the 1# saccharification tank 1, and the valve g is closed after conveying is completed.

When the enzyme preparation tank 2 and its pipe are to be cleaned, water is added to the enzyme preparation tank 2 (the volume of the added water must be greater than or equal to a volume of the conveying pipe), and pumped by the diaphragm pump 12 to the 1# saccharification tank 1 and water is further added (the volume of the added water must be greater than or equal to a volume of the conveying pipe) and pumped by the diaphragm pump 12 to the 1# saccharification tank 1 for cleaning the enzyme preparation tank 2 and the enzyme preparation pipe.
(I) After completion of the step (H), stirring reaction is performed for the 1# saccharification tank 1 for ≥4h and stirring is stopped after the reaction to wait for discharging bottoms. Based on the production situations, bottoms discharge time is reasonably arranged with specific bottoms discharge operations shown in the steps (J) and (K). Same operations are performed for 2# saccharification tank 1' for bottoms discharge.
(J) When discharging the bottoms of the 1# saccharification tank 1, the discharge valves of other tanks are closed, and the discharge valve b of the 1# saccharification tank is opened, and the sugar liquid containing protein is enabled to run through the second plate heat exchanger to cool down through heat exchange with the evaporation feed (pre-evaporation liquid), and enter the plate and frame filter 7 for filtration, protein is retained in the plate and frame filter 7, and a filtrate is conveyed into the buffer tank 8, and then into the rotary drum filter 10 for filtration to obtain a rotary drum filtrate, and decolorization, ion exchange and evaporation are performed for the rotary drum filtrate to finally obtain a finished ultrahigh maltose syrup, and the bottoms are discharged completely to the liquid level 0%.
(K) After completion of the step (J), the discharge valve b is closed and the valve e is opened to continue discharging other saccharified sugar liquids. The 1# saccharification tank 1 waits for feed at the same time. Air ejection, water ejection and air ejection are performed for cleaning the plate and frame filter 7 respectively for 10 to 15min, 2 to 5min and 15 to 20min, which are adjustable depending on a wetness of the protein residues. After the protein is dried, the protein is discharged to the spiral conveyor 11 below the plate and frame filter 7 for recovery.

During a filtration process of the plate and frame filter 7, when a flow rate is not satisfied or a pressure is >0.4MPa, the plate and frame filter 7 is to be changed.

### Embodiment 1: taking one saccharification tank as example

As shown in FIG. 1, there are two saccharification tanks and one (i.e. 1# saccharification tank 1) of the two saccharification tanks is taken as an example in the embodiment.
(11) a feed valve a of the 1# saccharification tank (volume 260m3) was opened, stirring was started and the liquefaction liquid to be saccharified with a temperature of 60°C, a pH of 5.0 and a DE value of 3.5 was allowed to enter the 1# saccharification tank 1, and the saccharifying enzyme was added at the rate of 0.32kg/dry basis ton at the same time. When the liquid level of the 1# saccharification tank1 was 100%, the feed valve a was closed.

The saccharifying enzyme includedβ-amylase (Maiteer biology M-100), pullulanase (novozymes Promozyme D6), maltogenic amylase (novozymes Maltogenase 2XL) which were blended at a weight ratio of 1:1:1.
(12) After completion of the step (11), stirring reaction was performed for the 1# saccharification tank 1 for 45h, and the 1# saccharification tank 1 was stood for 5h.
(13) After completion of the step (12), a discharge valve b of the 1# saccharification tank 1 was opened and the sugar liquid pump 6 was started to enable the sugar liquid to pass through the second plate heat exchanger to cool down through heat exchange with an evaporation feed (pre-evaporation liquid) and enter the plate and frame filter 7 for filtration, a filtrate was conveyed into the buffer tank 8 and then into the rotary drum filter 10 for filtration to obtain a rotary drum filtrate, and then decolorization, ion exchange and evaporation were performed for the rotary drum filtrate to finally obtain a finished ultrahigh maltose syrup.
(14) When the remaining liquid level of the 1# saccharification tank 1 was 15%, the discharge valve b was closed to stop discharging.
(15) After completion of the step (14), 1kg of lysophospholipase was weighed into the enzyme preparation tank 2, the diaphragm pump 12 was started and a valve g was opened to convey the lysophospholipase into the 1# saccharification tank 1 and then the valve g was closed.

When the enzyme preparation tank 2 and its pipe are to be cleaned, 10L of water was added to the enzyme preparation tank 2 and pumped by the diaphragm pump 12 to the 1# saccharification tank 1 and another 10L of water was added and pumped by the diaphragm pump 12 to the 1# saccharification tank 1 for cleaning the enzyme preparation tank 2 and the enzyme preparation pipe.

The lysophospholipase was novozymes Finizym^{®} W lysophospholipase.
(16) After completion of the step (15), stirring reaction was performed for the 1# saccharification tank 1 for 2h and stirring was stopped after the reaction.
(17) After completion of the step (16), a valve c and a valve h were opened, and the circulation pump 5 was started to enable the sugar liquid in the 1# saccharification tank 1 to run through the first plate heat exchanger for being heated cyclically. An openness of a vapor valve was 35% and a discharge temperature of the 1# plate heat exchanger was set to 95°C. When an entire temperature of the 1# saccharification tank 1 reached 95°C, cyclic heating was stopped.
(18) After completion of the step (17), 2kg of high-temperature-resistant α-amylase was weighed into the enzyme preparation tank, the diaphragm pump 12 was started and the valve g was opened to convey the high-temperature-resistant α-amylase into the 1# saccharification tank 1, and the valve g was closed after conveying is completed.

When the enzyme preparation tank 2 and its pipe are to be cleaned, 10L of water was added to the enzyme preparation tank 2 and pumped by the diaphragm pump 12 to the 1# saccharification tank 1 and another 10L of water was added and pumped by the diaphragm pump 12 to the 1# saccharification tank 1 for cleaning the enzyme preparation tank 2 and the enzyme preparation pipe.

The high-temperature-resistant α-amylase was Dupont SPEZYME^{®} POWERLIQ with its activity ≥30099LU/g.
(19) After completion of the step (18), stirring reaction was performed for the 1# saccharification tank 1 for 4h and stirring was stopped after the reaction.
(110) After completion of the step (19), the discharge valve b of the 1# saccharification tank 1 was opened, the sugar liquid containing protein was enabled to run through the second plate heat exchanger to cool down through heat exchange with the evaporation feed (pre-evaporation liquid), and enter the plate and frame filter 7 for filtration, protein was retained in the plate and frame filter 7, and a filtrate was conveyed into the buffer tank 8, and then into the rotary drum filter 10 for filtration to obtain a rotary drum filtrate, decolorization, ion exchange and evaporation were performed for the rotary drum filtrate to finally obtain a finished ultrahigh maltose syrup, and bottoms were completely discharged to the liquid level 0%.
(111) After completion of the step (110), the discharge valve b was closed, and air ejection, water ejection and air ejection were performed on the plate and frame filter for 10min, 3min and 15min respectively. After the protein was dried, the protein was discharged to the spiral conveyor 11 below the plate and frame filter 7 for recovery.

### Control embodiment1: taking one saccharification tank as example

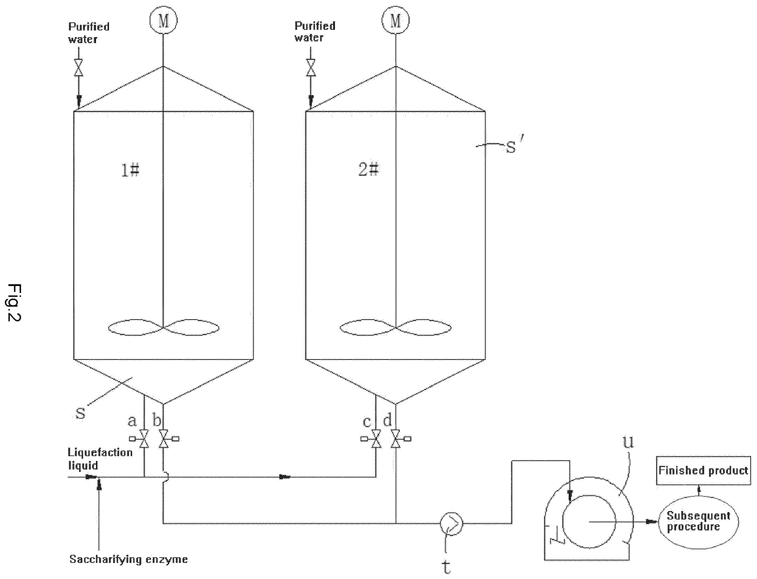

As shown in FIG. 2, there are two saccharification tanks: 1# saccharification tank s and 2# saccharification tank s' and one (i.e. 1# saccharification tank s) of the two saccharification tanks is taken as an example in the control example.
(d1) A feed valve a of the 1# saccharification tank s(volume 260m3) was opened, stirring was started and the liquefaction liquid to be saccharified with a temperature of 60°C, a pH of 5.0 and a DE value of 3.5 was allowed to enter the 1# saccharification tank s, and the saccharifying enzyme was added at the rate of 0.32kg/dry basis ton at the same time. When the liquid level of the saccharification tank was 100%, the feed valve a was closed.

The saccharifying enzyme included β-amylase (Maiteer biology M-100), pullulanase (novozymes Promozyme D6), maltogenic amylase (novozymes Maltogenase 2XL) which were blended at a weight ratio of 1:1:1.
(d2) After completion of the step (d1), stirring reaction was performed for the 1# saccharification tank s for 45h, and then the 1# saccharification tank s was stood for 5h.
(d3) After completion of the step (d2), a discharge valve b of the 1# saccharification tank s was opened and a sugar liquid pump t was started to enable a sugar liquid to pass through a rotary drum filter u to obtain a rotary drum filtrate and decolorization, ion exchange and evaporation were performed for the rotary drum filtrate to finally obtain a finished ultrahigh maltose syrup. All was discharged to the liquid level 0%.

In the control embodiment1, the rotary drum filter uses diatomite as filter aid.

Diatomite consumptions and protein recovery amounts of the embodiment 1 and the control embodiment1 are shown in Table 1. In the embodiment, 1, the diatomite consumption is obviously decreased, the bottoms filtration of the plate and frame filter 7 is smooth, the column pressure is 0.1 to 0.2MPa, and the collected protein is lump-like without obvious water. Protein is easy to discharge and suitable for sales.

**Table 1**

| | Embodiment 1 (new process) | Control embodiment(original process) |
|---|---|---|
| Diatomite consumption (kg/dry basis ton) | 0.3 | 9.5 |
| Protein recovery amount(kg/dry basis ton) | 0.02 (lump-like and water content of 42%) | 0 |

The above descriptions are made to the preferred embodiments of the present invention shall not be intended to limit the present invention. Any modifications, equivalent substitutions and improvements made within the spirit and principle of the present invention shall fall within the scope of protection of the present invention.

## Claims

1. A system for recovering protein in a production process of an ultrahigh maltose syrup, comprising a saccharification tank, an enzyme preparation tank, a first plate heat exchanger, a second plate heat exchanger, a plate and frame filter, a buffer tank and a rotary drum filter, wherein the enzyme preparation tank is communicated with a liquid inlet end of the saccharification tank through a pipe, and the liquid inlet end of the saccharification tank is communicated with a liquefaction liquid to be saccharified and a saccharifying enzyme through a pipe respectively, a liquid outlet end of the saccharification tank is communicated with an inlet end of the first plate heat exchanger through a pipe, an outlet end of the first plate heat exchanger is communicated with the liquid inlet end of the saccharification tank through a pipe, the liquid outlet end of the saccharification tank is further communicated with an inlet end of the second plate heat exchanger through a pipe, an outlet end of the second plate heat exchanger is communicated with an inlet end of the plate and frame filter through a pipe, residues filtered by the plate and frame filter contain protein to be recovered, a residue outlet end of the plate and frame filter is communicated with an inlet end of a spiral conveyor through a pipe, the residues containing protein is transferred by the spiral conveyor for recycling, a liquid outlet end of the plate and frame filter is communicated with a liquid inlet end of the buffer tank through a pipe, a liquid outlet end of the buffer tank is communicated with the rotary drum filter through a pipe, and a liquid outlet end of the rotary drum filter is communicated with a subsequent working procedure.

2. The system of claim 1, further comprising: a circulation pump, a sugar liquid pump and a syrup pump, wherein the circulation pump is disposed on the pipe communicating the first plate heat exchanger with the liquid outlet end of the saccharification tank, the sugar liquid pump is disposed on the pipe communicating the second plate heat exchanger with the liquid outlet end of the saccharification tank, and the syrup pump is disposed on the pipe communicating the rotary drum filter with the liquid outlet end of the buffer tank.

3. A method of recovering protein in a production process of an ultrahigh maltose syrup, using the system for recovering protein in a production process of an ultrahigh maltose syrup according to claim 1 or 2, wherein one or more saccharification tanks are used in the system; wherein,
when one saccharification tank is used, the method of recovering protein in a production process of an ultrahigh maltose syrup comprises the following steps:
(a) opening a feed valve a of the 1# saccharification tank, starting stirring and allowing the liquefaction liquid to be saccharified to enter the 1# saccharification tank, and adding the saccharifying enzyme at the same time; when the liquid level of the 1# saccharification tank is 100%, closing the feed valve a;
(b) after completion of the step (a), performing stirring reaction for the 1# saccharification tank until the stirring reaction ends, and standing the 1# saccharification tank to make protein float at an upper part of the 1# saccharification tank as possible;
(c) after completion of the step (b), opening a discharge valve b of the 1# saccharification tank and starting the sugar liquid pump to enable the syrup to pass through the second plate heat exchanger to cool down through heat exchange with an evaporation feed and enter the plate and frame filter for filtration, conveying a filtrate into the buffer tank and then into the rotary drum filter for filtration to obtain a rotary drum filtrate, and then performing decolorization, ion exchange and evaporation for the rotary drum filtrate to finally obtain a finished ultrahigh maltose syrup;
(d) when the remaining liquid level of the 1# saccharification tank is 10-15%, closing the discharge valve b to stop discharging;
(e) after completion of the step (d), weighing a lysophospholipase into the enzyme preparation tank, starting a diaphragm pump and opening a valve g to convey the lysophospholipase into the 1# saccharification tank and closing the valve g;
(f) after completion of the step (e), performing stirring reaction for the 1# saccharification tank until the stirring reaction ends;
(g) after completion of the step (f), opening a valve c and a valve h and starting the circulation pump, enabling the syrup in the 1# saccharification tank to run through the first plate heat exchanger for being heated cyclically; when an entire temperature of the 1# saccharification tank reaches a preset temperature, stopping cyclic heating;
(h) after completion of the step (g), weighing a high-temperature-resistant α-amylase into the enzyme preparation tank, starting the diaphragm pump and opening the valve g to convey the high-temperature-resistant α-amylase into the 1# saccharification tank, and closing the valve g;
(i) after completion of the step (h), performing stirring reaction for the 1# saccharification tank until the stirring reaction ends;
(j), after completion of the step (i), opening the discharge valve b of the 1# saccharification tank, enabling the sugar liquid containing protein to run through the second plate heat exchanger to cool down through heat exchange with the evaporation feed, and enter the plate and frame filter for filtration, retaining protein in the plate and frame filter, and conveying a filtrate into the buffer tank, and then into the rotary drum filter for filtration to obtain a rotary drum filtrate, and performing decolorization, ion exchange and evaporation for the rotary drum filtrate to finally obtain a finished ultrahigh maltose syrup, discharging bottoms completely to the liquid level 0%;
(k) after completion of the step (j), closing the discharge valve b and after the protein is dried, discharging the protein to the spiral conveyor below the plate and frame filter for recovery;
when a plurality of saccharification tanks, for example, two saccharification tanks, are used, the method of recovering protein in a production process of an ultrahigh maltose syrup comprises the following steps:
(A) opening the feed valve a of the 1# saccharification tank, starting stirring, and allowing the liquefaction liquid to be saccharified to enter the 1# saccharification tank and adding the saccharifying enzyme at the same time; when the liquid level of the 1# saccharification tank is 100%, closing the feed valve a and opening the valve d to allow the liquefaction liquid to be saccharified and the saccharifying enzyme to enter the 2# saccharification tank;
(B) after completion of the step (A) and closing of the feed valve a, performing stirring reaction for the 1# saccharification tank until the stirring reaction ends, and standing the 1# saccharification tank to make protein float at an upper part of the 1# saccharification tank as possible; and performing same operations for the 2# saccharification tank;
(C), after completion of the step (B), and standing of the 1# saccharification tank, opening the discharge valve b of the 1# saccharification tank and starting the sugar liquid pump to enable the syrup to pass through the second plate heat exchanger to cool down through heat exchange with an evaporation feed and enter the plate and frame filter for filtration, conveying a filtrate into the buffer tank and then into the rotary drum filter for filtration to obtain a rotary drum filtrate, and then performing decolorization, ion exchange and evaporation for the rotary drum filtrate to finally obtain a finished ultrahigh maltose syrup;
(D) when the remaining liquid level of the 1# saccharification tank is 10-15%, stop discharging; firstly closing the discharge valve b and then opening a valve e, and performing the same discharge operations to the 2# saccharification tank as to the 1# saccharification tank; during a discharge process of the 2# saccharification tank, performing enzyme preparation reaction treatment for the bottoms of the 1# saccharification tank by referring to the following specific operations shown in the steps (E), (F), (G), (H) and (I); performing the same operations for the bottoms of the 2# saccharification tank for enzyme preparation reaction treatment;
(E) weighing a lysophospholipase into the enzyme preparation tank, starting the diaphragm pump and opening the valve g to convey the lysophospholipase into the 1# saccharification tank and closing the valve g;
(F) after completion of the step (E), performing stirring reaction for the 1# saccharification tank until the stirring reaction ends;
(G) after completion of the step (F), opening the valve c and the valve h and starting the circulation pump, enabling the syrup in the 1# saccharification tank to run through the first plate heat exchanger for being heated cyclically; when an entire temperature of the 1# saccharification tank reaches a preset temperature, stopping cyclic heating;
(H) after completion of the step (G), weighing a high-temperature-resistant α-amylase into the enzyme preparation tank, starting the diaphragm pump and opening the valve g to convey the high-temperature-resistant α-amylase into the 1# saccharification tank, and closing the valve g after completing the conveying;
(I) after completion of the step (H), performing stirring reaction for the 1# saccharification tank until the stirring reaction ends with the bottoms to be discharged by referring to the specific operations shown in the steps (J) and (K); performing the same operations to discharge the bottoms of the 2# saccharification tank;
(J) when discharging the bottoms of the 1# saccharification tank, closing the discharge valves of other tanks, and opening the discharge valve b of the 1# saccharification tank, enabling the sugar liquid containing protein to run through the second plate heat exchanger to cool down through heat exchange with the evaporation feed, and enter the plate and frame filter for filtration, retaining protein in the plate and frame filter, and conveying a filtrate into the buffer tank, and then into the rotary drum filter for filtration to obtain a rotary drum filtrate, and performing decolorization, ion exchange and evaporation for the rotary drum filtrate to finally obtain a finished ultrahigh maltose syrup, discharging the bottoms completely to the liquid level 0%;
(K) after completion of the step (J), closing the discharge valve b and opening the valve e, and continuing discharging other saccharified syrups; being ready to feed the 1# saccharification tank at the same time; after the protein is dried, discharging the protein to the spiral conveyor below the plate and frame filter for recovery.

4. The method of claim 1, wherein, in the steps (a) and (A), the liquefaction liquid to be saccharified has a temperature of 58 to 61°C, a pH of 5.0 to 5.4 and a DE value of 3.0 to 4.0.

5. The method of claim 1, wherein, in the steps (a) and (A), the addition amount of the saccharifying enzyme is 0.30 to 0.35kg/dry basis ton.

6. The method of claim 1, wherein, in the steps (b) and (B), the stirring reaction of the 1# saccharification tank lasts for 40 to 45h which is determined based on the addition amount of the saccharifying enzyme, and the standing proceeds for≥5h; in the steps (f) and (F), the stirring reaction is≥2h; in the steps (i) and (I), the stirring reaction is≥4h.

7. The method of claim 1, wherein, in the steps (e) and (E), the lysophospholipase is added based on 0.16 to 0.22kg/dry basis ton.

8. The method of claim 1, wherein, in the steps (g) and (G), the preset temperature is 95 to 97°C.

9. The method of claim 1, wherein, in the steps (h) and (H), the high-temperature-resistant α-amylase is added based on 0.36 to 0.44kg/dry basis ton.

10. The method of claim 1, wherein, in the steps (k) and (K), air ejection, water ejection and air ejection last respectively for 10 to 15min, 2 to 5min and 15 to 20min, which are adjustable depending on a wetness of the protein residues.
